# EUROPEAN PATENT APPLICATION

(11) **EP 2 371 346 A1**
(43) Date of publication of application: **05.10.2011**
(21) Application number: 11002598.8
(22) Date of filing: 29.03.2011
(51) Int. Cl.: A61K 6/083

(54) **Paste-type polymerizable composition**

(30) Priority: 31.03.2010 JP 2010082223
(71) Applicant: GC Corporation, Tokyo 113-0033 (JP)
(72) Inventor: Yarimizu, Hideki, Tokyo (JP); Tanaka, Koji, Tokyo (JP); Hokii, Yusuke, Tokyo (JP)
(74) Representative: Müller-Boré & Partner Patentanwälte

(57) **Abstract**

To provide a paste-type polymerizable composition used in a tooth restoration treatment, where the paste-type polymerizable composition has excellent storing stability, has proper polymerization property in the presence of water, and hardly discolors with passage of time after polymerization; the paste-type polymerizable composition consists of a first paste and a second paste, wherein the first paste includes an ascorbic acid compound (b), (meth)acrylate (d) not having an acid group and a filler (e) not reacting to acid, and the second paste includes a peroxide (a) not having a polymer structure and being compatible with water or dissoluble with water, (meth)acrylate (c) having an acid group, (meth)acrylate (d) not having an acid group, a filler (e) not reacting to acid and water (f).

## Description

The present invention relates to a paste-type polymerizable composition used in a tooth restoration treatment, where the paste-type polymerizable composition has excellent storage stability, has proper polymerization property in a presence of water, and hardly discolors with passage of time after polymerization.

As a polymerization catalyst for polymerizing a paste-type composition including a monomer, an oligomer, and a prepolymer of acrylate and/or methacrylate which radically polymerize, a combination of an organic peroxide and an aromatic tert-amine has been traditionally used (e.g., refer to Japanese Patent Application Laid-Open No. S62-246514). In this combination, the amounts of the organic peroxide and the aromatic tert-amine which are blended with the paste-type composition are adjusted, and a polymerization inhibitor is used together, whereby a polymerization curing time can be controlled, and the paste-type composition before polymerization can have storing stability.

However, the organic peroxide is an unstable material having a half-life period. Thus, when a great amount of the organic peroxide has been blended with the paste-type composition before polymerization in order to quicken the polymerization curing time, the paste-type composition itself easily gelates before polymerization at a time of being stored for a long time. By contrast, when a great amount of the polymerization inhibitor has been blended with the paste-type composition before polymerization, there is aproblemthat the polymerization curing time at a time of use of the composition comes to be extremely long. Therefore, for storing the paste-type composition for a long period of time, a countermeasure that the paste-type composition is stored under refrigeration to thereby delay deterioration of the organic peroxide must be taken. Further, since a reducing agent such as the aromatic tert-amine discolors the composition to a yellowish color after polymerization, there is a problem in the tooth restoration treatment in which the color is aesthetically important. Furthermore, when the combination of the organic peroxide and the aromatic tert-amine is used under a condition of much water content, such as in an oral cavity, there is a problem that sufficient effect cannot be obtained.

As a method to prevent the composition after polymerization from discoloring, a paste-type polymerizable composition containing ternary catalysts consisting of a pyrimidinetrione derivative, an organometallic compound, and an organohalogen compound has been disclosed (e.g., refer to Japanese Patent Application Laid-Open No. 2003-105008). Since none of these ternary catalysts has a half-life period, the polymerization curing time is stable even when the composition is stored for a long period of time. However, the composition can not have sufficient polymerization property under the condition of much water content, such as in an oral cavity.

As methods for improving the storing stability, a dental composition combining hydroperoxide, a thiourea derivative, and a copper compound (e.g., refer to Japanese Patent Application Laid-Open No. 2007-056020), a dental composition using a hydrogen peroxide-polyvinyl pyrrolidone composite (e.g., refer to Japanese Patent Application Laid-Open No. 2008-088086) and the like have been disclosed. However, these dental compositions cannot have proper storing stability for a long period of time, and cannot have sufficient polymerization curability under the condition of much water content, such as in an oral cavity.

An objective is to provide a paste-type polymerizable composition used in a tooth restoration treatment, where the paste-type polymerizable composition has excellent storing stability, has proper polymerization property in the presence of water, and hardly discolors after polymerization.

Accordingly, present inventors carried out earnest works to solve the aforementioned problems and, as a result, they found out the followings to complete the present invention. When a peroxide not having a polymer structure and ascorbic acid are used, solubility with respect to water hardly varies, and evaporation of water with passage of time hardly occurs. Further, even when (meth) acrylate having a hydrophilic group such as -OH group in a molecule is blended, compatibility after kneading is stable.

Even when a paste-type polymerizable composition is not refrigerated for being stored, the paste-type polymerizable composition has excellent storing stability, has proper polymerization property in the presence of water, and hardly discolors after polymerization.

More specifically, according to an aspect of the present invention, a paste-type polymerizable composition includes a peroxide (a) not having a polymer structure and being compatible with water or dissoluble with water, an ascorbic acid compound (b), (meth) acrylate (c) having an acid group, (meth)acrylate (d) not having an acid group, a filler (e) not reacting to acid, and water (f).

It is preferable that the paste-type polymerizable composition is configured to have a first paste and a second paste, where the first paste includes the ascorbic acid compound (b), the (meth)acrylate (d) not having an acid group, and the filler (e) not reacting to acid, and the second paste includes the peroxide (a) not having a polymer structure and being compatible with water or dissoluble with water, the (meth)acrylate (c) having an acid group, the (meth)acrylate (d) not having an acid group, the filler (e) not reacting to acid, and water (f). Furthermore, it is more preferable that the first paste includes a filler (g) reacting to acid, and the second paste includes polycarboxylic acid (h).

As the peroxide (a) not having a polymer structure and being compatible or dissoluble with water, which is used in the present invention, potassiumperoxodisulfate, sodium peroxodisulfate, ammonium peroxodisulfate, tert-butyl hydroperoxide, stearoyl peroxide, succinic acid peroxide, and the like can be used. These can be used by mixing two or more kinds. Particularly, potassium peroxodisulfate and tert-butyl hydroperoxide are preferable because of having solubility with water and excellent polymerization property.

The ascorbic acid compound (b) could be L(+)-ascorbic acid, L(+)-calcium ascorbate, L(+)-sodium ascorbate, dehydroascorbic acid, isoascorbic acid, sodium isoascorbate, (+)-5,6-0-isopropylidene-L-ascorbic acid, 2,6-di-0-palmitoyl-L-ascorbic acid, 6-0-palmitoyl-L-ascorbic acid, D-araboascorbic acid, or the like. Particularly, sodium isoascorbate is preferable because of having stability in the composition. In addition, these ascorbic acid compounds can be salt hydrates. Furthermore, these compounds can be used by mixing two or more kinds.

The (meth) acrylate compound (c) having an acid group is cured by polymerization reaction and comes to be a part of base materials of the composition. Simultaneously, the (meth)acrylate compound (c) having an acid group has an effect for giving to a tooth-adhesive composition an adhesive property with respect to ceramics such as zirconia, alumina, and the like or an alloy containing noble metals, which are materials used for dental restorations. The (meth) acrylate compound in the present invention means various kinds of monomers, oligomers, and prepolymers of acrylate or methacrylate compounds. As the (meth) acrylate compound having an acid group, (meth) acrylate having one or more phosphate groups or carboxyl groups in one molecule is preferable. Since the phosphate group has stronger acidity than the carboxyl group, the phosphate group has a high effect for dissolving a smear layer of a tooth surface and for tooth decalcification. Particularly, the phosphate group can exercise an effect for improving adhesive property to enamel.

The (meth) acrylate compound having a phosphate group can be 2-(meth)acryloyloxyethyldihydrogen phosphate, bis[2-(meth)acryloyloxyethyl]hydrogen phosphate, 2-(meth)acryloyloxyethylphenylhydrogen phosphate, 6-(meth)acryloyloxyhexyldihydrogen phosphate, 6-(meth)acryloyloxyhexylphenylhydrogen phosphate, 10-(meth)acryloyloxydecyldihydrogen phosphate, 1, 3-di(meth)acryloylpropane-2-dihydrogen phosphate, 1, 3-di(meth)acryloylpropane-2-phenylhydrogen phosphate, bis[5-{2-(meth)acryloyloxyethoxycarbonyl} heptyl]hydrogen phosphate, or the like. Particularly, 10-(meth)acryloyloxydecyldihydrogen phosphate is preferable because of having excellent adhesive property and self-stability of an acrylate compound. These (meth)acrylate compounds having the phosphate group can be used independently or by mixing two or more kinds.

The (meth) acrylate compound having a carboxyl group can be 4-(meth)acryloxyethyltrimellitic acid, 4-(meth)acryloxyethyltrimellitic acid anhydride, 4-(meth)acryloxydecyltrimellitic acid, 4-(meth)acryloxydecyltrimellitic acid anhydride, 11-(meth)acryloyloxy-1,1-undecanedicarboxylicacid,1, 4-di(meth)acryloyloxypyromellitic acid, 2-(meth)acryloyloxyethylmaleic acid, 2-(meth)acryloyloxyethylphthalic acid, 2-(meth)acryloyloxyethylhexahydrophthalic acid, or the like. Particularly, 4-(meth)acryloxyethyltrimellitic acid and 4-(meth)acryloxyethyltrimellitic acid anhydride are preferable because of having an excellent adhesive property. These (meth)acrylate compounds having a carboxyl group can be used by mixing two or more kinds.

The (meth) acrylate compound (d) not having an acid group can be methyl (meth) acrylate, ethyl (meth)acrylate, isopropyl (meth)acrylate, n-butyl (meth)acrylate, isobutyl (meth)acrylate, hydroxypropyl (meth)acrylate, tetrahydrofurfryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-methoxyhexyl (meth)acrylate, 2-ethylhexyl (meth)acrylate, benzyl (meth)acrylate, 2-hydroxy-1,3-di(meth)acryloxypropane, ethylene glycol di(meth)acrylate, diethylene glycol di(meth)acrylate, triethylene glycol di(meth)acrylate, butylene glycol di(meth)acrylate, neopentyl glycol di(meth)acrylate, 1, 3-butanediol di(meth)acrylate, 1, 4-butanediol di(meth)acrylate, 1, 6-hexanediol di (meth) acrylate, trimethylolpropane tri (meth) acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, bisphenol A glycidyl (meth) acrylate or the like. Amonomer, oligomer, and prepolymer of these compounds can be properly used. Further, as for (meth) acrylate having urethane bond, di-2-(meth)acryloxyethyl-2, 2, 4-trimethylhexamethylene dicarbamate, 1, 3, 5-tris [1, 3-bis{(meth)acryloyloxy}-2-propoxycarbonylaminohexan e]-1, 3, 5-(1H, 3H, 5H) triazine-2, 4, 6- trione, and 2, 2-bis-4-(3-(meth)acryloyloxy-2-hydroxypropyl)-phenyl propane, can be used. In addition, the (meth)acrylate having urethane bond can be (meth)acrylate of urethane oligomer including 2, 2'-di(4-hydroxycyclohexyl) propane, 2-oxypanone, hexamethylene diisocyanate, and 2-hydroxyethyl (meth)acrylate, and (meth)acrylate of urethane oligomer including 1, 3-butanediol, hexamethylene diisocyanate, and 2-hydroxyethyl (meth)acrylate. These (meth)acrylates can be used independently or by mixing two or more kinds.

The filler (e) not reacting to acid is blended to increase strength of the paste-type polymerizable composition. More specifically, the filler (e) not reacting to acid can be powders of anhydrous silicic acid, glasses such as barium glass, alumina glass, potassium glass, and the like, feldspar, fumed silica, hydrous silicic acid, quartz, and the like. In order to chemically bond with (meth) acrylate, such the filler can be subjected to a surface treatment with a silane coupling agent, such as γ-methacryloxypropyltrimethoxysilane, vinyltrichlorosilane, vinyltriethoxysilane, vinyltrimethoxysilane, vinyltriacetoxysilane, vinyltri (methoxyethoxy) silane, or the like. Further, a organic and inorganic composite filler, which is produced by previously mixing the aforementioned filler with (meth)acrylate compound, polymerizing/curing the mixture, and pulverizing the cured body, can be used. Particularly, anhydrous silicic acid and hydrous silicic acid have an effect for preventing the paste-type polymerizable composition before polymerization from gelling even when being stored for a long period of time. These fillers are used independently or by mixing two or more.

Water (f) is blended for dissolving and activating the peroxide (a) not having a polymer structure and being compatible with water or dissoluble with water.

In the paste-type polymerizable composition according to the present invention, since the storing property decreases when the ascorbic acid compound (b) and the (meth) acrylate (c) having an acid group coexist, it is preferable that the ascorbic acid compound (b) and the (meth)acrylate (c) having an acid group are blended separately.

For example, the paste-type polymerizable composition preferably consists of a first paste and a second paste, where the first paste includes the ascorbic acid compound (b), the (meth)acrylate (d) not having an acid group and the filler (e) not reacting to acid, and the second paste includes the peroxide (a) not having a polymer structure and being compatible with water or dissoluble with water, the (meth)acrylate (c) having an acid group, the (meth)acrylate (d) not having an acid group, the filler (e) not reacting to acid and water (f).

Furthermore, the paste-type polymerizable composition according to the present invention preferably includes a filler (g) reacting to acid in the first paste, and polycarboxylic acid (h) in the second paste. The filler (g) reacting to acid is a filler for generating a cement reaction with the (meth) acrylate compound having an acid group and the polycarboxylic acid (h) in the composition, in the presences of the water (f). It is necessary that the filler (g) reacting to acid is blended separating from the (meth)acrylate (c) having an acid group and the polycarboxylic acid (h) . More particularly, fluoroaluminosilicate glass powder or synthetic zeolite powder can be used.

Preferably, the polycarboxylic acid (h) is a copolymer or a homopolymer including one or more kinds selected from acrylic acid, methacrylic acid, 2-chloroacrylic acid, 3-chloroacrylic acid, aconitic acid, mesaconic acid, maleic acid, itaconic acid, fumaric acid, glutaconic acid, and citraconic acid, does not include a polymerizable and ethylenically unsaturated double bond, and has a weight average molecular weight of 5, 000 to 40, 000. If the weight averagemolecularweight is less than 5, 000, the strength of a cured body decreases easily, and the adhesive strength to a tooth tends to decrease. If the weight average molecular weight is more than 40,000, operativity tends to decrease.

In the paste-type polymerizable composition according to the present invention, a photopolymerization initiator, a thickener, a pigment, a stabilizer, and an antimicrobial agent, which are generally used, can be blended according to necessity.

The present invention will be described in detail below using examples, but the present invention is not limited in the examples.

### [Preparation of the first paste and the second paste]

The blending ratios of the first paste and the second paste used in each of examples and comparative examples are shown in Table 2. In experiments, 1g of the first paste and 1g of the second paste were weighed and taken on a kneading paper, and kneaded for 40 seconds by using a spatula to thereby uniformly mix the pastes, and the uniformly mixed pastes were used.

Brevity codes in Table 2 are as follows.
IA acid Na: sodium isoascorbate
IA acid: isoascorbic acid
p-amine: p- tolyldiethanolamine
N-AcTU: N-acetylthiourea
AcCu: acetylacetone copper
UDMA: di-2-methacryloxyethyl-2, 2, 4-trimethylhexamethylene dicarbamate
TEGDMA: triethylene glycol dimethacrylate
HEMA: 2-hydroxyethyl methacrylate
CLVX-S: high-purity crystalline quartz filler having a particle diameter of 5 µm or less (produced by Tatsumori Co., Ltd.)
AS-A200: hydrophilic fumed silica (produced by Nippon Aerosil Corporation)
Glass I: fluoroaluminosilicate glass powder I (the preparation method will be described below)
Glass II: fluoroaluminosilicate glass powder II (the preparation method will be described below)
CQ: camphorquinone
BHT: butylhydroxytoluene
tert-BHPO: tert-butyl hydroperoxide
KPS: potassium peroxodisulfate
BPO: benzoyl peroxide
CHPO: cumene hydroperoxide
4-META: 4-methacryloxyethyltrimellitic acid anhydride MDP: 2-methacryloyloxyethyldihydrogen phosphate
PAA: Polyacrylic acid

### [Preparation of fluoroaluminosilicate glass powders]

Blending ratios of the fluoroaluminosilicate glass powders I and II (Glass I and Glass II in Table 2) as the filler (g) reacting to acid are shown in Table 1.

**[Table 1]**

| Unit : g | Fluoroaluminosilicate glass powders | |
|---|---|---|
| | I | II |
| Aluminum oxide | 21 | 23 |
| Anhydrous silicic acid | 44 | 41 |
| Calcium fluoride | 12 | 10 |
| Calcium phosphate | 14 | 13 |
| Strontium carbonate | 9 | 13 |

The fluoroaluminosilicate glass powder I were produced by fully mixing raw materials, holding the mixture in a high temperature electric furnace at 1200°C for 5 hours so as to melt a glass, cooling the melted glass, pulverizing the glass for 10 hours using a ball mill, and sieving the pulverized glass with a 200 mesh sieve(ASTM). The fluoroaluminosilicate glass powder II were produced by a process similar to that of the fluoroaluminosilicate glass powder I excepting that the glass was heated at 1100°C to melt.

### <Curing time>

According to the curing time test in JIS T6609-2:2005 (Dentistry-Water-basedcements - Part In : Resin-modified cements) 5. 5, the curing time was measured. However, the curing time was measured at 23°C in order to easily detect a difference of curabilities due to a difference of the blending ratios.

Further, the curing time was similarly measured after the samples of examples and comparative examples were stored for 1 year at a room temperature (23°C) . These results were shown in Table 2.

### <Bending test>

According to the bending test in JIS T6609-2:2005 (Dentistry-Water-based cements - Part In : Resin-modified cements) 5. 12, the flexural strength was measured. These results were shown in Table 2.

### <Discoloring confirmation test>

According to the color tone stability in JIS T6514:2005 (dental composite resin for filling) 4.4, samples were soaked in distilled water at 37°C for 7 days, and a color tone change (ΔE) of the samples before and after soaking was measured by using a spectrocolorimeter (the product name: CM-3610d, produced by Konica Minolta Sensing Inc.). These results were shown in Table 2. In this case, lower ΔE indicates the lower color tone change.

**[Table 2]**

| | | | | Examples | | | | | | Comparative examples | | | |
|---|---|---|---|---|---|---|---|---|---|---|---|---|---|
| | | | | 1 | 2 | 3 | 4 | 5 | 6 | 1 | 2 | 3 | 4 |
| First Paste | (b) | Ascorbic acid compound | IA acid Na | 1 | | 1 | | 1 | 1 | | | | |
| | | | IA acid | | 1 | | 1 | | | | | | |
| | (b') | Other polymerization initiators | p-amine | | | | | | | 1 | 0.5 | | |
| | | | N-AcTU | | | | | | | | | 1 | 1 |
| | | | AcCu | | | | | | | | | 0.1 | 0.1 |
| | (d) | (Meth)acrylate not having an acid group | UDMA | 29 95 | 29 95 | 29 65 | 29 65 | 10 95 | 10 65 | 29 95 | 7 95 | 29 85 | 7 35 |
| | | | TEGDMA | 10 | 10 | 10 | 10 | 11 | 11 | 10 | 8 | 10 | 8 |
| | | | HEMA | 6 | 6 | 6 | 6 | 22 | 22 | 6 | 18 | 6 | 18 |
| | (e) | Filler not reacting to acid | CLVX-S | 48 | 48 | 48 | 48 | 5 | 5 | 48 | | 48 | |
| | | | AS-A200 | 5 | 5 | 5 | 5 | 5 | 5 | 5 | 5 | 5 | 5 |
| | (g) | Filler reacting to acid | Glass I | | | | | 45 | | | 60 | | |
| | | | Glass II | | | | | | 45 | | | | 60 |
| | Z | Others | CQ | | | 0 3 | 0 3 | | 0 3 | | | | |
| | | | BHT | 0 05 | 0 05 | 0 05 | 0 05 | 0 05 | 0 05 | 0 05 | 0 05 | 0 05 | 0 05 |
| | Total | | | 100 | 100 | 100 | 100 | 100 | 100 | 100 | 100 | 100 | 100 |
| Second Paste | (a) | Peroxide being compatible with water or dissoluble with water | tert-BHPO | 0 5 | | | 0 5 | 0.5 | | | | | |
| | (a') | Other peroxides | BPO | | | | | | | 0 5 | 0 5 | | |
| | | | Cumene hydroperoxide | | | | | | | | | 0 5 | 0 5 |
| | (c) | (Meth)acrylate having an acid group | 4-META | 8 | | 8 | | 5 | | 8 | | 8 | |
| | | | MDP | | 5 | | 5 | | 2 | | 5 | | 5 |
| | (d) | (Meth)acrylale not having an acid group | UDMA | 12 | 13 | 12 | 13 | 4 | 4 | 12 | 4 | 12 | 4 |
| | | | TEGDMA | 12 | 13 | 12 | 13 | 5 | 5 | 12 | 5 | 12 | 5 |
| | | | HEMA | 15 | 16 | 15 | 16 | 10 | 13 | 15 | 10 | 15 | 10 |
| | (e) | Filler not reacting to acid | CLVX-S | 38 | 38 | 38 | 38 | 15 | 15 | 38 | 15 | 38 | 15 |
| | | | AS-A200 | 5 | 5 | 5 | 5 | 1 | 1 | 5 | 1 | 5 | 1 |
| | (f) | Water | Water | 9 45 | 9 45 | 9 45 | 9 45 | 29 45 | 29 45 | 9 45 | 29 45 | 9 45 | 29 45 |
| | (h) | Polycarboxylic acid | PAA | | | | | 30 | 30 | | 30 | | 30 |
| | Z | Other | BHT | 0 05 | 0 05 | 0 05 | 0 05 | 0 05 | 0 05 | 0 05 | 0 05 | 0 05 | 0 05 |
| | Total | | | 100 | 100 | 100 | 100 | 100 | 100 | 100 | 100 | 100 | 100 |
| | | | | | | | | | | | | | |
| | Curing time JIS T6609-2·2005 | | | 5 minutes 00 second | 6 minutes 30 seconds | 5 minutes 00 second | 6 minutes 30 seconds | 4 minutes 30 seconds | 4 minutes 30 seconds | Not cured | 20 minutes 00 second | Not cured | 15 minutes 00 second |
| | Flexural strength (MPa) | | | 65 | 56 | 72 | 63 | 35 | 40 | Cannot be measured | 17 | Cannot be measured | 20 |
| | Discoloring confirmation test ΔE | | | 0 8 | 1 4 | 0 6 | 1 2 | 1 0 | 1 1 | Cannot be measured | 4 3 | Cannot be measured | 3 7 |

## Claims

1. A paste-type polymerizable composition comprising:
(a) a peroxide not having a polymer structure and being compatible with water or dissoluble with water;
(b) an ascorbic acid compound;
(c) (meth)acrylate having an acid group;
(d) (meth)acrylate not having an acid group;
(e) a filler not reacting to acid; and
(f) water.

2. The paste-type polymerizable composition as claimed in claim 1,
wherein the paste-type polymerizable composition comprises a first paste and a second paste,
wherein the first paste comprises:
(b) the ascorbic acid compound;
(d) the (meth)acrylate not having an acid group; and
(e) the filler not reacting to acid, and
wherein the second paste comprises:
(a) the peroxide not having a polymer structure and being compatible with water or dissoluble with water;
(c) the (meth)acrylate having an acid group;
(d) the (meth)acrylate not having an acid group;
(e) the filler not reacting to acid; and
(f) water.

3. The paste-type polymerizable composition as claimed in claim 2,
wherein the first paste further comprises (g) a filler reacting to acid, and
wherein the second paste further comprises (h) polycarboxylic acid.
